# EUROPEAN PATENT APPLICATION

(11) **EP 3 476 349 A1**
(43) Date of publication of application: **01.05.2019**
(21) Application number: 17198008.9
(22) Date of filing: 24.10.2017
(51) Int. Cl.: A61B 18/20, A61B 18/00

(54) **A CUTTING ELEMENT FOR USE IN A HAIR CUTTING DEVICE**

(71) Applicant: Koninklijke Philips N.V., 5656 AE Eindhoven (NL)
(72) Inventor: Jurna, Martin, 5656 AE Eindhoven (NL); Verhagen, Rieko, 5656 AE Eindhoven (NL); Boamfa, Marius Iosif, 5656 AE Eindhoven (NL); Thumma, Kiran Kumar, 5656 AE Eindhoven (NL)
(74) Representative: de Haan, Poul Erik

(57) **Abstract**

There is provided a cutting element (2) for use in a hair cutting device (10) for cutting hair on a body of a subject, the cutting element comprising: an optical waveguide (4) for coupling to a light source (14) to receive light, wherein the optical waveguide is for contacting hair during a hair cutting operation; a support structure (6) for supporting the optical waveguide (4), wherein a portion (18) of the side wall along at least part of the length of the optical waveguide (4) is in contact with the support structure (6); and a biasing element (8) for applying a biasing force to the optical waveguide (4) and/or at least a part of the support structure (6) to maintain contact between the portion (18) of the side wall of the optical waveguide (4) and the support structure (6).

## Description

### FIELD OF THE INVENTION

The invention relates to a cutting element for use in a hair cutting device, and in particular relates to a cutting element for use in a hair cutting device that uses light to cut or shave hair.

### BACKGROUND OF THE INVENTION

Shaving devices for cutting and shaving hair on a body of a subject typically make use of cutting elements comprising one or more blades that cut hairs as the blade is moved across the skin of the subject. The blades can be static within the device, for example as in a wet razor, whereas in other types of devices, for example electric shavers, one or more blade elements can be actuated (e.g. rotated or oscillated) in order to produce a cutting action.

However, an alternative type of shaving device has been proposed in WO 2014/143670 that makes use of laser light. In particular a laser light source is provided that is configured to generate laser light having a wavelength selected to target a predetermined chromophore to effectively cut a hair shaft. A fiber optic is located on a shaving portion of the device that is positioned to receive the laser light from the laser light source at a proximal end, conduct the laser light from the proximal end toward a distal end, and emit the light out of a cutting region of the fiber optic and toward hair when the cutting region is brought in contact with the hair.

### SUMMARY OF THE INVENTION

To achieve good shaving effectiveness, the cutting element of the shaving device (i.e. the fiber optic in the case of the device in WO 2014/143670) needs to be brought very close to the skin and the laser light needs to have sufficient power to cut the hair through melting.

In particular, high intensity laser light is required in the fiber optic to facilitate the initiation of hair cutting. However, the high laser intensity results in high temperatures on the region of the fiber optic where hair cutting (melting) occurs, which is seen experimentally to reach a temperature close to or higher than the glass transition temperature, Tg, of the fiber (in the case of fibers formed from a glass or glass-liked material). This is especially the case when low wavelength light (around 450nm) is used for the cutting, as this is strongly absorbed by (darker) hair and as such concentrates the build-up of heat at the position close to the fiber optic. One of the major causes of breakage of the fiber optic is the excessive heat generated during the cutting process (e.g. above the Tg of the fiber) which may cause an increase in mechanical tension due to the attachment between the fiber optic and the hair cutting device. In addition, during a hair cutting operation hairs can get stuck to the fiber optic which increases the likelihood of mechanical breakage of the fiber optic, and the mechanical impact between hair and the fiber optic during the hair cutting operation may also provide additional unwanted tension of the fiber optic. In the free air fiber configuration for laser shaving, the fiber is thin, weak and the fiber cutting section is suspended in free air. The mechanical impact between hair and fiber provides additional tension to the fiber and could easily lead to mechanical breakage. Furthermore the quality of the fiber degrades after increased hair cutting, making the fiber more brittle, sticky and micro tapered at hot spot locations.

One way to address these problems is to provide a support structure for supporting the optical fiber along at least part of the length of the fiber. However, due to differences between the thermal expansion coefficient of the optical fiber and the support structure, there can be increases in tension in the optical fiber (for example if the support structure expands more quickly than the optical fiber) or loosening (air gaps) between the optical fiber and the support structure (for example if the optical fiber expands more quickly than the support structure), both of which can lead to breaking of the optical fiber.

To better address one or more of these concerns, in a first aspect there is provided a cutting element for use in a hair cutting device, the cutting element comprising: an optical waveguide for coupling to a light source to receive light, wherein the optical waveguide is for contacting hair during a hair cutting operation; a support structure for supporting the optical waveguide , wherein a portion of the side wall along at least part of the length of the optical waveguide is in contact with the support structure; and a biasing element for applying a biasing force to the optical waveguide and/or at least a part of the support structure to maintain contact between the portion of the side wall of the optical waveguide and the support structure.

In some embodiments, the biasing element may be for applying the biasing force to compensate for a difference in thermal expansion of the optical waveguide and the support structure during the hair cutting operation.

In some embodiments, at least one end of the optical waveguide may be fixedly attached to the support structure.

In some embodiments, the support structure may comprise a first portion and a second portion, one end of the optical waveguide being attached to the first portion and an opposite end of the optical waveguide being attached to the second portion, and the biasing element may be configured to apply the biasing force to the first portion and the second portion to bias the first portion and the second portion towards each other or away from each other.

In some embodiments, the biasing element may comprise a spring element. In these embodiments, the spring element may be at least one of a leaf spring, a coiled spring, a bar spring, and a spiral spring.

In some embodiments, the cutting element further comprises a layer of low refractive index material between the portion of the side wall of the optical waveguide and the support structure. In these embodiments, the layer may have a refractive index that is lower than that of the optical waveguide. Moreover, in these embodiments, the layer may be at least 100nm thick.

In some embodiments, the support structure may comprise a surface for supporting the optical waveguide during the hair cutting operation, where the surface may be shaped such that the optical waveguide follows a straight or non-straight path along the surface.

In some embodiments, the support structure may comprise a groove configured to receive the portion of the side wall of the optical waveguide. In these embodiments, the groove may have a shape corresponding to that of the optical waveguide.

In some embodiments, the support structure may be made of at least one of metal, ceramic, glass, and crystal.

In some embodiments, the optical waveguide may be an optical fiber.

According to a second aspect of the invention, there is provided a hair cutting device for cutting hair on a subject, the hair cutting device comprising: a light source for generating light at one or more specific wavelengths corresponding to wavelengths absorbed by one or more chromophores in or on hair; and a cutting element according to the first aspect, wherein the optical waveguide of the cutting element is coupled to the light source to receive light.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a block diagram of a cutting element for use in a hair cutting device according to an embodiment of the invention;
Fig. 2 is a schematic drawing showing different perspective views of an exemplary cutting element according to an embodiment of the invention;
Fig. 3 is a schematic drawings showing a perspective view of an exemplary hair cutting device according to an embodiment of the invention; and
Fig. 4 is a graph illustrating the refractive index of hair.

### DETAILED DESCRIPTION OF THE EMBODIMENTS

As noted above, the present invention provides an improvement in the durability of the cutting element, in particular an optical waveguide (e.g. a fiber optic) in the cutting element, during operation, and this can be achieved by adopting an arrangement which provides a biasing force to the optical waveguide to maintain contact between the optical waveguide and a support structure during a hair cutting operation. The support structure provides support to the optical waveguide along most or all of its length of the part of the optical waveguide intended to be brought into contact with hairs during use, so that the length of the optical waveguide is supported as the optical waveguide is brought into contact with hairs. The biasing element provides a biasing force so that the optical waveguide continues to be supported by the support structure along at least part (or most) of its length during the hair cutting operation despite changes in the length of the optical waveguide and/or support structure due to thermal expansion. Where the relative thermal expansion properties of the optical waveguide and support structure mean that the optical waveguide expands at a faster rate than the underlying support structure during heating, the biasing element provides that the optical waveguide can be kept in contact with the support structure along the length of the optical waveguide despite the additional length of the optical waveguide. Where the relative thermal expansion properties of the optical waveguide and support structure mean that the support structure expands at a faster rate than the optical waveguide during heating, the biasing element provides that the optical waveguide can be kept in contact with the support structure along the length of the optical waveguide without the optical waveguide being subjected to high stretching or strain forces due to the relative increase in length of the underlying support structure.

It will be appreciated that the invention is applicable for use in shaving devices (e.g. razors or electric shavers), and any other type of device that is used to cut hair (e.g. hair clippers), even if those devices do not necessarily aim to provide a 'clean shave' (i.e. to remove hair at the level of the skin).

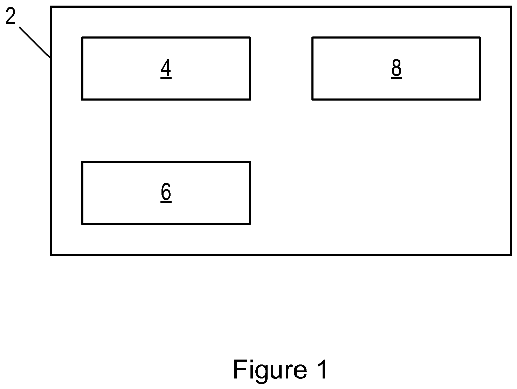

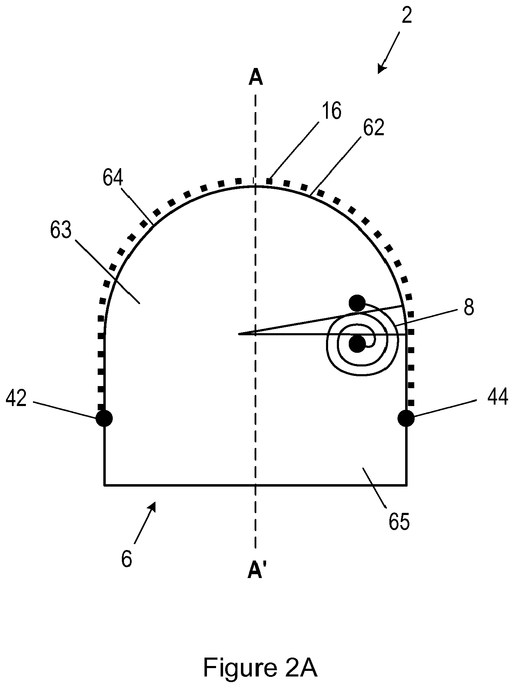

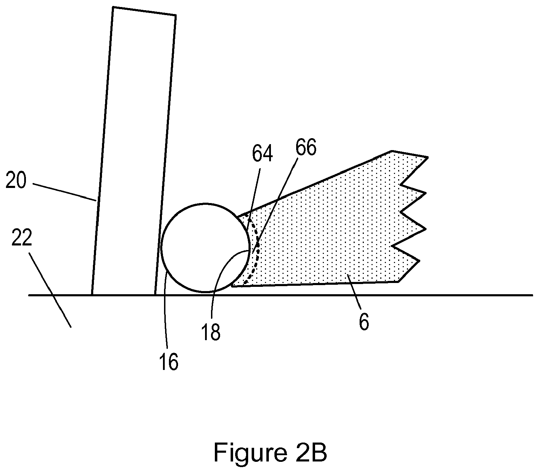

Fig. 1 is a block diagram of a cutting element 2 according to an embodiment of the invention, and Fig. 2 shows different perspectives of a cutting element 2 according to an exemplary embodiment of the invention. Specifically, Fig. 2A shows a bottom view of the cutting element 2 and Fig. 2B shows a cross-sectional view of the cutting element 2 along line A-A' of Fig. 2A. The cutting element 2 can be used in a hair cutting device to cut or shave hair on a body of a subject. The subject may be a person or an animal. The hair may be facial hair (i.e. hair on the subject's face), or hair on the subject's head or other part of their body (legs, chest, etc.).

The cutting element 2 comprises an optical waveguide 4 that is supported by a support structure 6, so that the optical axis of the optical waveguide 4 (i.e. the line along which light typically propagates through the optical waveguide 4) is generally perpendicular to the direction in which the hair cutting device (and thus cutting element 2) is moved across the surface of the skin of a subject. In this way, hairs contact a cutting face 16 formed by a first portion of a side wall along at least part of the length of the optical waveguide 4 as the hair cutting device is moved across the skin of the subject. A second portion 18 of the side wall along at least part of the length of the optical waveguide 4 is in contact with the support structure 6 such that the optical waveguide 4 is supported by the support structure 6. The cutting face (the first portion) of the optical waveguide 4 can be understood as the 'front' of the optical waveguide 4 as it faces the direction of movement when the hair cutting device is being used to cut hair, and the second portion 18 can be understood as the 'back' of the optical waveguide 4 that faces away from the direction of movement. The cutting face 16 and the second portion 18 are generally along the same part of the length of the optical waveguide 4 so the second portion 18 can also be understood as being 'behind' some or all parts of the cutting face 16. In this way, the support structure 6 is in contact with parts of the optical waveguide 4 that are behind the cutting face 16, which enables the support structure 6 to directly support the optical waveguide 4/cutting face 16 at locations where the optical waveguide 4 impinges on hairs.

The optical waveguide 4, in particular the first portion of the side wall of the optical waveguide 4, is shown in Fig. 2B in contact with a hair 20 and close to, but not in contact with, the skin 22. The optical waveguide 4 preferably has a circular or generally circular cross-section, and the cutting face 16 can extend at least 180° around the circumference of the optical waveguide 4. In some embodiments, the cutting face 16 preferably extends up to 270° around the circumference of the optical waveguide 4.

The optical waveguide 4 can be made from any suitable material or combination of materials. For example optical waveguides can be composed of comprise a glass or glass-liked material, such as silica, fluoride glass, phosphate glass, chalcogenide glass, crown glass (such as BK7), quartz, sapphire, yttrium aluminium garnet, YAG, yttrium aluminosilicates, aluminosilicates, and/or oxyfluorides. It will be appreciated that a glass or glass-liked material is a material that has a glass transition temperature at which the material changes from a solid 'glass' state into a viscous state, and which is below the melting temperature of the crystalline state of the material if one exists. In case the material of the optical waveguide 4 is crystalline, then the relevant temperature is the melting temperature rather than a glass transition temperature. In such alternative embodiments, the optical waveguide 4 can be composed of or comprise a non-glass-like material, i.e. a material that does not have a glass transition temperature. Examples of suitable materials include crystalline sapphire and crystalline YAG.

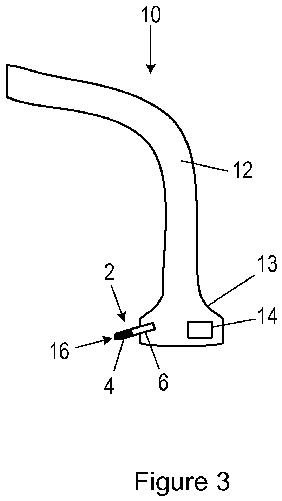

The optical waveguide 4 can be coupled to a light source in a hair cutting device to receive light when the cutting element 2 is being used in a hair cutting device. Hair is cut by light coupling into the hair from the optical waveguide 4 through frustrated total internal reflection, which is explained in further detail below with reference to Fig. 3.

In some embodiments, the optical waveguide 4 is an optical fiber, although those skilled in the art will be aware of other types of optical waveguide that can be used according to the invention, such as a slab waveguide, a strip waveguide, or a photonic crystal waveguide. An optical fiber comprises a core, and in some embodiments also comprises a cladding, which may or may not fully encompass the core (e.g. part of the core may be exposed). In some embodiments, the optical waveguide 4 does not include any cladding around its core. However it will be appreciated that in some embodiments the optical waveguide 4 can comprise cladding around the core of the optical waveguide, although preferably no cladding is present along the cutting face 16 (and indeed, in some embodiments the cutting face 16 can correspond to those parts of the optical waveguide 4 where there is no cladding).

The optical waveguide 4 is arranged along a surface 62 of the support structure 6. The support structure 6 therefore provides mechanical stability and support to at least a part of the optical waveguide 4. The support structure 6 may be formed from at least one of a metal material, a glass material, a crystal material, and a ceramic material, i.e. the support structure 6 may be formed of one of metal, ceramic, glass, crystal, or a combination of any of metal, ceramic, glass, and crystal. In some embodiments, such as that shown in Fig. 2B, the support structure 6 can comprise a groove 64 configured to receive the second portion 18 of the side wall along the length of the optical waveguide 4. The groove 64 may have a shape corresponding to that of the optical waveguide 4 so as to secure the optical waveguide 4 at the support structure 6 against lateral movement, without necessarily having to fixedly attach the whole of the optical waveguide 4 to the support structure 6. For example, in some embodiments where the optical waveguide 4 comprises a tapering portion, the groove 64 may be shaped so as to specifically correspond to the tapering portion of the optical waveguide 4. In some embodiments, the groove 64 has a 'v' cross-section or a curved cross-section (e.g. a 'c' cross section). In some embodiments, at least a part of the optical waveguide 4 can be fixedly attached to at least a part of the support structure 6. For example, the optical waveguide 4 can be welded (e.g. laser welded) to the support structure 6 and/or clamped to the support structure 6. In some embodiments, one or both of the ends of the optical waveguide 4 are attached to the support structure 6.

Also, as shown in Fig. 2B, the cutting element 2 can comprise a layer 66 of low refractive index material between the second portion 18 of the side wall of the optical waveguide 4 (i.e. the portion which is in contact with the support structure 6) and the support structure 6. This layer 66 acts to prevent the leakage of light from the optical waveguide 4 into the support structure 6. Preferably, the material of this layer 66 has a low refractive index in sense that the refractive index is lower than that of the optical waveguide 4 (and in particular lower than the part of the optical waveguide 4 that is in contact with the support structure 6, e.g. the cladding). The material of this layer 66 can have a refractive index lower than 1.0. In some embodiments, the layer 66 can be formed from silver and/or aluminium. In some embodiments, the layer 66 of lower refractive index material may be at least 100nm thick. In addition, in some embodiments, an additional layer (not shown in the drawing) of quartz may be provided so as to protect the layer 66 of low refractive index material from oxidation, especially when the layer 66 is a metallic layer. This additional layer may be much thinner than the layer 66, for example the additional layer might by just a few nm thick. In some embodiments, the layer 66 of low refractive index material is applied to the optical waveguide 4, whereas in other embodiments the layer 66 may be applied to the support structure 6. In some embodiments, the layer 66 of low refractive index material is a coating.

In some embodiments, the cutting element 2 is configured such that the size of the contact area between the optical waveguide 4 and the support structure 6 is minimised, so as to reduce loss of light from the optical waveguide 4 due to imperfect reflection between the layer 66 and the optical waveguide 4. This is because the reflection of light from metals such as silver is not 100%, but rather around 98%-99% (due to the imaginary part of the refractive index), and the losses due to multiple reflections in an optical waveguide 4 (e.g. multiple reflections per mm of optical waveguide 4) add up quickly if the surface size of the area of the layer 66 at the second portion 18 of the side wall of the optical waveguide 4 is not minimised. The support structure 6 therefore preferably provides support along most or all of the length of the optical waveguide 4, while providing a small contact area along that length.

The support structure 6 comprises a surface 62 for supporting the optical waveguide 4 during the hair cutting operation. In some embodiments the surface 62 (and thus the support structure 6) is shaped such that the optical waveguide 4 follows a non-straight path along the surface 62. In particular, in the present embodiment shown in Fig. 2A (which may be a bottom view or a top view of the cutting element 2), the surface 62 has a curved shape such that the optical waveguide 4 also follows a corresponding curved path along the surface 62. It will be appreciated in other embodiments the surface 62 and the optical waveguide 4 may adopt other arrangements and shapes depending on a number of factors, such as dimensions of the cutting element 2 or of the hair cutting device to which the cutting element 2 is attached. The surface 62 can also be referred to as a 'front' surface 62 of the cutting element 2 since it faces the direction of movement of the cutting element 2 during a cutting operation. In one of these other embodiments, the surface 62 (and thus the support structure 6) is shaped so that the optical waveguide 4 follows a straight path along the surface 62 (i.e. the surface 62 itself is straight). In the embodiments where the optical waveguide 4 follows a straight path, the support structure 6 may include the groove 64 and/or layer 66 of low refractive index material.

It will be appreciated that although the exemplary cutting element 2 in Fig. 2 comprises both a groove 64 and layer 66 of low refractive index material, a cutting element 2 could include just one of the groove 64 and layer 66. Some exemplary cutting elements 2 do not include a groove 64 or layer 66.

The cutting element 2 further comprises a biasing element 8 which is configured to apply a biasing force to the optical waveguide 4 and/or at least part of the support structure 6 to maintain contact between the second portion 18 of the side wall of the optical waveguide 4 and the support structure 6 during a hair cutting operation. The biasing element 8 can be for applying the biasing force in order to compensate for differences in thermal expansion of the optical waveguide 4 and the support structure 6 during the hair cutting operation. Generally, the biasing element 8 is used to maintain a level of tension in the optical waveguide 4 that keeps the optical waveguide 4 flat against (i.e. in contact with) the support structure 6, despite relative differences in thermal expansion of the optical waveguide 4 and support structure 6 as the optical waveguide 4 and support structure 6 are heated during a hair cutting operation. The biasing element 8 can apply the biasing force directly to the optical waveguide 4, or it can apply the biasing force to the support structure 6 or a part of the support structure 6 to which the optical waveguide 4 is attached. In some embodiments, the biasing element 8 is a spring element, and in these embodiments, the spring element may preferably be a constant force spring.

In the embodiment shown in Fig. 2A, the biasing element 8 is positioned between a first portion 63 of the support structure 6 and a second portion 65 of the support structure 6. The first portion 63 and the second portion 65 may be separated by a slit or other opening that can allow relative motion of the first portion 63 and the second portion 65, for example due to thermal expansion of the support structure 6 and/or optical waveguide 4, and/or due to the action of the biasing element 8. One end 42 (herein referred to as a "first end") of the optical waveguide 4 is attached to the first portion 63 of the support structure, and an opposite end 44 (herein referred to as a "second end") of the optical waveguide 4 is attached to the second portion 65 of the support structure.

The biasing element 8 is configured or arranged to apply the biasing force to the first portion 63 and the second portion 65 to bias the first portion 63 and the second portion 65 towards each other or away from each other, depending on the arrangement of the components of the cutting element 2 as well as the nature and/or material properties of the optical waveguide 4 and the support structure 6. For example, during the hair cutting operation differences in thermal expansion of the optical waveguide 4 and the support structure 6 may cause the length of the optical waveguide 4 to increase more than the length of the surface 62 supporting the optical waveguide 4, which then causes the optical waveguide 4 to separate from the support structure 6, e.g. forming an air gap, or increases the tension in the attachment between the optical waveguide 4 and support structure 6. Either way, the optical waveguide 4 is thus at an increased risk of mechanical breakage. In these embodiments, the biasing element 4 may be configured to applying the biasing force so as to bias the first portion 63 and the second portion 65 away from each other so as to accommodate this difference in the increase of the length of the optical waveguide 4 and the corresponding length along the surface 62 of the support structure 6. As another example, during the hair cutting operation differences in thermal expansion of the optical waveguide 4 and the support structure 6 may cause the length of the surface 62 supporting the optical waveguide 4 to increase more than the length of the optical waveguide 4, which then causes tension in the optical waveguide 4 as it is stretched by the optical waveguide 4. The optical waveguide 4 is thus at an increased risk of mechanical breakage. In these embodiments, the biasing element 4 may be configured to applying the biasing force so as to bias the first portion 63 and the second portion 65 towards each other so as to accommodate this difference in the increase of the length of the surface 62 supporting the optical waveguide 4 relative to the increase in length along the surface 62 of the support structure 6.

As described above the biasing element can be a spring element. Although it is described above that in some embodiments the spring element may preferably be a constant force spring, in other embodiments the spring element may be one of a compression spring, an extension spring, and a torsion spring. Moreover, in some embodiments, the spring element may be implemented as a coiled spring, a leaf spring, a bar spring, and a spiral spring. It will be appreciated that in other embodiments the biasing element may be any type of element which is capable of providing a biasing force, such as elements or materials capable of storing elastic energy.

Fig. 3 shows a cutting element 2 being used in a hair cutting device 10 that is in the form of a handheld razor according to an exemplary embodiment. Fig. 3 shows a side view of the razor 10. The razor 10 comprises a handle 12 for the subject (or other user of the device 10) to hold, and head portion 13 to which the cutting element 2 can be attached or with which the cutting element 2 is integrated.

A light source 14 is provided in the hair cutting device 10 that generates light (e.g. laser light) at one or more specific wavelengths. When the cutting element 2 is attached to or integrated with the hair cutting device 10, the light source 14 is optically coupled to the optical waveguide 4 so that the laser light generated by the light source 14 is coupled into the optical waveguide 4 (and specifically coupled into an end of the optical waveguide 4 so that the laser light propagates through the optical waveguide 4). In some embodiments, the power (optical power) of the light generated by the light source 14 can be controlled for example by a control unit (not shown in the drawings). In some embodiments, a light source 14 can be coupled to opposite ends of the optical waveguide 4 so that light is input to the optical waveguide 4 at both ends.

The light source 14 is configured to generate laser light at one or more specific wavelengths that can be used to cut or burn through hair. In particular, each wavelength can correspond to the wavelength of light absorbed by a chromophore that is found in or on hair. As is known, a chromophore is the part of a molecule that provides the molecule with its colour. Thus, the laser light will be absorbed by the chromophore and converted into heat which will melt or burn the hair or otherwise destroy the bonds in the molecules of the hair, and it is this melting or burning that provides the cutting action of the cutting element 2.

Suitable chromophores that can be targeted by the laser light generated by the light source 14 include, but are not limited to, melanin, keratin, and water. Suitable wavelengths of laser light that can be used include, but are not limited to, wavelengths selected from the range 380nm (nanometers) to 500nm and 2500nm to 3500nm. Those skilled in the art will be aware of the wavelengths of light that are absorbed by these chromophores, and thus also the specific wavelengths of light that that light source 14 should generate for this purpose, and further detailed are not provided herein.

In some embodiments the light source 14 can be configured to generate laser light at a plurality of wavelengths (either simultaneously or sequentially), with each wavelength being selected to target a different type of chromophore. This can improve the cutting action of the optical waveguide 4 since multiple types of molecules in the hair may be burnt using the laser light. Alternatively multiple light sources 14 can be coupled to a respective optical waveguide 4 at the cutting element 2.

As noted above, when the hair cutting device 10 is operated with the light source 14 outputting light at a light intensity that is sufficiently high to melt hairs, the heating of the hair also heats part of the optical waveguide 4 in contact with the hair, and this heating can increase the temperature of the optical waveguide 4 and consequentially the temperature of at least a part of the support structure 6. The increase in temperature at the optical waveguide 4 and the support structure 6 would result in respectively different thermal expansion of these components of the cutting element 2. Since the optical waveguide 4 and the support structure are usually made of different materials with different thermal properties (e.g. different thermal expansion coefficients), there are differences in thermal expansion of these components which, when not accommodated for, would increase the tension built up at the optical waveguide 4 and therefore the likelihood of mechanical breakage. Thus, as explained above with reference to Fig. 2, the biasing element 8 is provided so as to apply a biasing force to the optical waveguide 4 to maintain contact between the second portion of the side wall of the optical waveguide 4 and the support structure 6 during the hair cutting operation while compensating for this difference in thermal expansion between the optical waveguide 4 and the support structure 6. Accordingly, the durability of the cutting element 2 can be improved.

In some embodiments, the light source 14 may be controlled by a control unit (not shown in the drawing) that controls the operation of the hair cutting device 10. In particular, the activation and deactivation of the light source 14 may be controlled by the control unit, for example in response to an input from a user of the hair cutting device 10. The control unit can comprise one or more processors, processing units, multi-core processors or modules that are configured or programmed to control the hair cutting device 10. The control unit can also comprise or be associated with a memory or memory module that stores data and computer readable code that is configured to be executed by the control unit to cause the control unit and the hair cutting device 10 to cut hair.

Although the light source 14 is shown as being incorporated into the head portion 13, it will be appreciated that the position of the light source in the hair cutting device 10 as shown in Fig. 3 is not limiting. Likewise it will be appreciated that the embodiments shown in Figs. 2 and 3 are merely examples, and the optical waveguide 4, the support structure 6 and the biasing element 8 can be incorporated or used in place of a conventional blade in any type of hair cutting device 10 that conventionally comprises a blade for physically cutting or slicing hair (whether the blade is static or actuated in order achieve a cutting action).

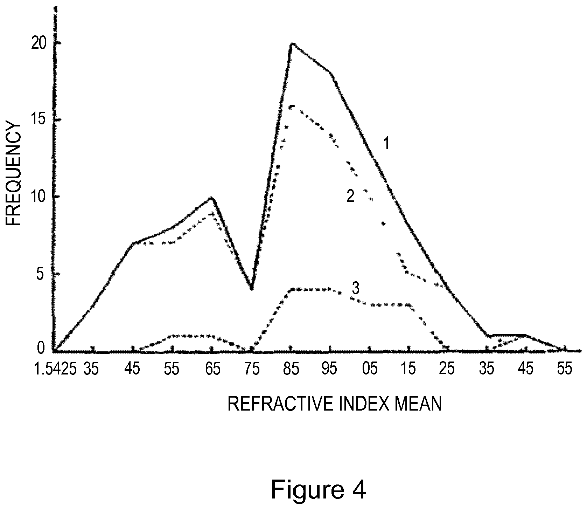

The graph in Fig. 4 illustrates the refractive index of hair, which can be found in a paper by M. D. Greenwell, A. Willner, Paul L. Kirk: Human Hiar Studies: III. Refractive Index of Crown Hair, 31 Am. Inst. Crim. L. & Criminology 746 (1940-1941). Curve 1 is a composite line, curve 2 is a line representing the refractive index for Caucasian people, and curve 3 is a line representing the refractive index for non-Caucasian people. Thus, it can be seen that the refractive index of hair is between (approximately) 1.545 and 1.555, although there will be variation between individuals. For example the above paper also recognises that the refractive index of hair can depend on the sex of the subject, e.g. the refractive index of hair on a female is generally higher than the refractive index of hair on a male.

As is known, the optical waveguide 4 acts as a waveguide for the light coupled from the light source 14 through the occurrence of total internal reflection, since the refractive index of air is lower than that of the optical waveguide 4. However, if an object that is a refractive index higher than the optical waveguide 4 is put into contact with the optical waveguide 4, then the total internal reflection is 'frustrated' and light can couple from the optical waveguide 4 into that object, thus heating the object. Thus, in order for light to be coupled into a hair from the optical waveguide 4 (to provide the cutting action), the optical waveguide 4 must have the same or a lower refractive index than hair at the point at which hair contacts the optical waveguide 4. Thus, the optical waveguide 4 must have the same or a lower refractive index than hair at least at the cutting face 16 portion of the optical waveguide 4. Preferably the refractive index of the optical waveguide 4 at the cutting face 16 is the same as that of hair since that provides the best coupling of light from the optical waveguide 4 to the hair.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage.

Any reference signs in the claims should not be construed as limiting the scope.

## Claims

1. A cutting element (2) for use in a hair cutting device, the cutting element comprising:
an optical waveguide (4) for coupling to a light source (14) to receive light, wherein the optical waveguide (4) is for contacting hair during a hair cutting operation;
a support structure (6) for supporting the optical waveguide (4), wherein a portion (18) of a side wall along at least part of the length of the optical waveguide (4) is in contact with the support structure (6); and
a biasing element (8) for applying a biasing force to the optical waveguide (4) and/or at least a part of the support structure (6) to maintain contact between the portion (18) of the side wall of the optical waveguide (4) and the support structure (6).

2. A cutting element (2) as claimed in claim 1, wherein the biasing element (8) is for applying the biasing force to compensate for a difference in thermal expansion of the optical waveguide (4) and the support structure (6) during the hair cutting operation.

3. A cutting element (2) as claimed in claim 1 or claim 2, wherein at least one end of the optical waveguide (4) is fixedly attached to the support structure (6).

4. A cutting element (2) as claimed in any of the preceding claims, wherein the support structure (6) comprises a first portion (63) and a second portion (65), one end (42) of the optical waveguide being attached to the first portion (64) and an opposite end (44) of the optical waveguide being attached to the second portion (65), and the biasing element (8) is configured to apply the biasing force to the first portion (63) and the second portion (65) to bias the first portion (63) and the second portion (65) towards each other or away from each other.

5. A cutting element (2) as claimed in any of the preceding claims, wherein the biasing element (8) comprises a spring element.

6. A cutting element (2) as claimed in claim 5, wherein the spring element is at least one of a leaf spring, a coiled spring, a bar spring, and a spiral spring.

7. A cutting element (2) as claimed in any of the preceding claims, wherein the cutting element further comprises a layer (66) of low refractive index material between the portion (18) of the side wall of the optical waveguide and the support structure (6).

8. A cutting element (2) as claimed in claim 7, wherein the layer (66) has a refractive index that is lower than that of the optical waveguide (4).

9. A cutting element (2) as claimed in claim 7 or claim 9, wherein the layer (66) is at least 100nm thick.

10. A cutting element (2) as claimed in any of the preceding claims, wherein the support structure (6) comprises a surface (62) for supporting the optical waveguide (4) during the hair cutting operation, where the surface (62) is shaped such that the optical waveguide (4) follows a straight or non-straight path along the surface.

11. A cutting element (2) as claimed in any of the preceding claims, wherein the support structure (6) comprises a groove (64) configured to receive the portion (18) of the side wall of the optical waveguide.

12. A cutting element (2) as claimed in claim 11, the groove (64) has a shape corresponding to that of the optical waveguide (4).

13. A cutting element (2) as claimed in any of the preceding claims, wherein the support structure (6) is made of at least one of metal, ceramic, glass, and crystal.

14. A cutting element (2) as claimed in any of the preceding claims, wherein the optical waveguide (4) is an optical fiber.

15. A hair cutting device (10) for cutting hair on a subject, the hair cutting device comprising:
a light source (14) for generating light at one or more specific wavelengths corresponding to wavelengths absorbed by one or more chromophores in or on hair; and
a cutting element (2) as claimed in any of the preceding claims, wherein the optical waveguide (4) of the cutting element (2) is coupled to the light source (14) to receive light.
